# EUROPEAN PATENT SPECIFICATION

(11) **EP 1 597 216 B1**
(45) Date of publication and mention of the grant of the patent: **11.08.2010**
(21) Application number: 04713902.7
(22) Date of filing: 24.02.2004
(51) Int. Cl.: C07C 1/24, C07C 15/46, B01J 21/04, B01J 35/10

(54) **PROCESS FOR THE PREPARATION OF STYRENE**
VERFAHREN ZUR HERSTELLUNG VON STYROL
PROCEDE DE PREPARATION DE STYRENE

(30) Priority: 25.02.2003 EP 03251123
(43) Date of publication of application: 23.11.2005
(73) Proprietor: Shell Internationale Research Maatschappij B.V., 2596 HR Den Haag (NL)
(72) Inventor: VAN BROEKHOVEN, Johannes, Adrianus, Maria, NL-1031 CM Amsterdam (NL); MESTERS, Carolus, Matthias, Anna, Maria, NL-1031 CM Amsterdam (NL)
(86) International application number: PCT/EP2004/050191
(87) International publication number: WO 2004/076389

(56) References cited:
- WO-A-99/58480
- US-B1- 6 420 620

## Description

The present invention relates to a process for the preparation of styrene comprising the gas phase dehydration of 1-phenylethanol at elevated temperature in the presence of a dehydration catalyst.

A commonly known method for manufacturing styrene is the coproduction of propylene oxide and styrene starting from ethylbenzene. In general such process involves the steps of (i) reacting ethylbenzene with oxygen or air to form ethylbenzene hydroperoxide, (ii) reacting the ethylbenzene hydroperoxide thus obtained with propene in the presence of an epoxidation catalyst to yield propylene oxide and 1-phenylethanol (also known as α-phenylethanol or methylphenylcarbinol), and (iii) converting the 1-phenylethanol into styrene by dehydration using a suitable dehydration catalyst.

The use of alumina catalysts in the dehydration of 1-phenylethanol per se is well known in the art.

For instance, US-A-3,526,674 discloses the use of an alumina catalyst in the liquid phase dehydration of 1-phenylethanol into styrene, wherein said alumina catalyst suitably has a BET surface area of 40 to 250 m²/g and is used in finely divided form, i.e. in the form of particles having a particle size of 0.15 mm (100 mesh) or less.

US-A-3,658,928 discloses a process for the gas phase dehydration of 1-phenylethanol into styrene in the presence of controlled amounts of added steam and in the presence of a catalyst, which suitably is a commercially available alumina catalyst like Harshaw Al-0104.

Harshaw Al-0104 catalyst has a pore volume of about 0.35 ml/g. A dehydration process using alumina (aluminium oxide: Al₂O₃) catalysts especially suitable for such process has been disclosed in WO 99/58480. Use of these catalysts allows advantageous conversion of 1-phenylethanol into styrene without many of the disadvantages of the use of prior art catalysts. However, even use of these improved catalysts still leads to the formation of heavy by-products, typically up to 5% of oligomers and polymers of styrene. These heavy by-products do not further convert and therefore decrease the overall yield of desired styrene. Furthermore, these high-molecular weight by-products tend to occupy the pores of the catalyst, after which the catalyst cannot longer be used for converting 1-phenylethanol into styrene. This necessitates a regeneration step of the catalyst, which unfavourably adds to the costs of the process.

It is therefore an object of the present invention to find a catalyst for the gas phase dehydration of 1-phenylethanol into styrene, wherein styrene is obtained at improved selectivity and 1-phenylethanol conversion can be maintained at a high level for a longer time. This means that the catalyst needs to be regenerated less frequently.

Within the context of the present application the term "styrene" also embraces substituted styrenes, by which are meant styrenes containing one or more substituents bonded to the aromatic ring or to the vinyl group. Such substituents typically include alkyl groups, such as methyl or ethyl groups. Similarly, the term "1-phenylethanol" also embraces substituted 1-phenyl-ethanols having the same substituents as the corresponding substituted styrenes.

It was found that an alumina dehydration catalyst wherein the catalyst has a BET surface area of from 80 to 140 m²/g and a pore volume (Hg) of more than 0.65 ml/g is suitable for the preparation of styrene by a gas phase dehydration of 1-phenylethanol at elevated temperature while the 1-phenylethanol dehydration was maintained at a high level for a long time. Additionally, it was found that such process produced less heavy by-products than prior art catalysts.

The pore volume (Hg) of the catalyst for use in the present invention is more than 0.65 ml/g. Preferably, the pore volume is at most 1.0 ml/g. More specifically, the catalyst preferably has a pore volume (Hg) of from 0.75 to 0.85 ml/g.

The BET surface area can be measured in any way known to be suitable to someone skilled in the art. The expression pore volume (Hg) stands for the pore volume as measured with the help of mercury. Suitable methods for measuring the porosity with the help of mercury are also well known to someone skilled in the art.

Shaped alumina catalysts with the properties required for use in this invention can be prepared by procedures well known in the art, for example by extrusion of alumina or alumina precursor pastes, followed by calcination. Examples of alumina precursors are alumina hydrates like aluminium oxide trihydrate, Al₂O₃.3H₂O (also known as gibbsite or bayerite) and aluminium hydroxide AlOOH (also known as boehmite or pseudo-boehmite). These alumina precursors are transformed to alumina during the calcination process. Typically, in such a process, alumina powder or alumina precursor powder is first optionally mixed with a binder powder. Suitable binder materials include inorganic oxides like oxides of silicon, magnesium, titanium, aluminium, zirconium, and silicon-aluminium. The weight ratio of binder to alumina powders may be from 0 (no binder present) up to 90:10. Typically an extrudable mixture is prepared from the solids (alumina powders and optionally binder) and water by mixing and kneading the ingredients and passing this mixture into the extruder. Such extrudable mixture typically has a paste-like appearance. It is within the normal skills of those skilled in the art to optimize the mixing/kneading procedure to obtain an extrudable paste and to select the most appropriate extrusion conditions. Besides the alumina, optional binder and water, the extrusion paste will normally also comprise extrusion aids to improve the extrusion process. Such extrusion aids are known in the art and include, for instance, peptizing agents and flocculating agents. Peptizing agents facilitate a more dense packing of the particles in the extrusion mixture, while flocculating agents promote the inclusion of water. Suitable peptizing agents are known in the art and include monovalent inorganic acids (e.g. hydrochloric acid and nitric acid) and organic acids such as aliphatic monocarboxylic acids, acyclic monocarboxylic acids, and fatty acids. Suitable flocculating agents are also well known and include polyelectrolytes like those commercially available under the trade names NALCO and SUPERFLOC. Burnout materials may also be used to increase the porosity of the final extrudate. Examples of burnout materials are polyethylene oxide, methylcellulose, ethylcellulose, latex, starch, nut shells or flour, polyethylene or any of the polymeric microspheres or microwaxes.

A catalyst particularly suitable for use in this invention can be made from pseudo-boehmite (AlOOH). Such powder is commercially available from Criterion Catalyst Company.

The extrudable mixture or paste obtained as described above is then subjected to an extrusion treatment. This extrusion treatment can be performed by conventional extrusion techniques known in the art. At the outlet of the extruder an orifice is present, which gives the extruded mixture the selected shape when leaving the extruder. When aiming to obtain spherically shaped extrudates, the wet extrudate is first spheronized in a suitable spheronizing device after having left the extruder before being subjected to calcination. The catalyst particles may have any shape, including spherical, cylindrical, trilobal, quadrulobal, star-shaped, ring-shaped, cross-shaped etc. The green extrudates obtained as described above are then optionally dried and subsequently subjected to a calcination step. A catalyst of the desired properties could be obtained by drying extrudates at temperatures of from 100 to 140 °C for several hours and thereafter calcining at high temperature for several hours.

The dehydration of 1-phenylethanol into styrene according to the present invention is carried out in the gas phase at elevated temperature. The term "elevated temperature" preferably is any temperature above 150 °C. The preferred dehydration conditions to be applied are those normally applied and include reaction temperatures of from 210 to 330 °C, more preferably of from 280 to 320 °C, and most preferably about 300 °C, and pressures in the range of from 0.1 to 10 bar, most preferably about 1 bara.

In the process according to the present invention the catalyst described hereinbefore was observed to have a reaction selectivity to styrene of at least 96% at a conversion of at least 99%, whilst selectivities of 97% or higher at conversions of 99% and higher have been achieved. In this connection, reaction selectivity is defined as the number of moles of styrene formed per mole of precursor compounds converted to products. Similarly, selectivities to other compounds such as heavy-ends are defined as the number of moles of precursor compounds to heavy-ends per mole of precursor compounds converted to products. Conversion is defined as the overall conversion level of 1-phenylethanol as determined under test conditions, i.e. the mole percentage of 1-phenylethanol converted relative to the total number of moles of 1-phenylethanol present in the feed. Furthermore, the selectivity of the catalyst towards heavy by-products like oligomers and ethers is very low: selectivity towards ethers typically is less than 0.8%, more suitably less than 0.3%, whereas selectivity towards oligomers typically is less than 3% and preferably 2% or less.

The invention will now be illustrated by the following examples without limiting the scope of the invention to these particular embodiments.

### Example 1

A trilobe-shaped catalyst having the physical properties as indicated in Table I (Ex-1) was tested for dehydration performance in a microflow unit consisting of a 13 mm diameter plugflow reactor, 1-phenylethanol feed vaporization facilities and product vapour condensing facilities. As 1-phenylethanol feedstock was used a sample of the process stream to the styrene reactor system of a commercial Propylene Oxide/Styrene Monomer plant. The feedstock contained 81.2% of 1-phenylethanol, 10.6% of methylphenylketone and 2% of water. The remainder up to 100% consisted of impurities and (by)products of the preceding oxidation and epoxidation sections. The outlet stream of the micro flow unit was liquefied by condensation and the resulting two-phase liquid system was analyzed by means of gas chromatographic analysis.

The dehydration experiment was carried out at test conditions of 1.0 bara pressure and a temperature of 300 °C. The feed rate of 1-phenylethanol was maintained at 30 grams per hour and the reactor tube was loaded with 20 cm³ of catalyst. The reaction was continued for approximately 140 hours after which the experiment was stopped.

Activity (conversion) and reaction selectivity of the catalyst after 50 run hours were determined from the gas chromatographic analyses of reaction product samples. Activity after 120 hours was also measured. Data are reported in Table 1. Activity and selectivity have been defined above.

### Example 2

The procedure as described in Example 1 was repeated, except that a different sample of feedstock containing 81.3% of 1-phenylethanol and 9.9% of methylphenylketone was used. Data are reported in Table 1 (Ex-2).

### Comparison Example 1

The procedure as described in Example 1 was repeated, except that the trilobe-shaped catalyst had a BET surface area of 149 m². Physical properties are indicated in Table 1 (Comp-Ex-1). Only 1-phenylethanol conversion was monitored during the experiment, which was terminated after 98 hours, when 1-phenylethanol conversion was only 79%.

### Comparison Example 2

The procedure as described in Example 1 was repeated, except that a star-shaped catalyst with physical properties within the ranges as described in the process according to WO 99/58480 was used. The reaction was continued for approximately 120 hours. Activity and selectivity data are given in Table 1 (Comp-Ex-2).

### Comparison Example 3

The procedure as described in Example 1 was repeated, except that a trilobe-shaped catalyst with physical properties within the ranges as described in the process according to WO 99/58480 was used. A sample of feedstock containing 79.0% of 1-phenylethanol and 10.0% of methylphenylketone was used. Data are reported in Table 1 (Comp-Ex-3). The experiment was terminated after 113 hours, when 1-phenylethanol conversion was only 91%. Activity and selectivity data are given in Table 1 (Comp-Ex-3).

**TABLE I Catalyst properties and performance**

| | Ex-1 | Ex-2 | Comp-Ex-1 | Comp-Ex-2 | Comp-Ex-3 |
|---|---|---|---|---|---|
| Surface area (BET, m²/g) | 110 | 110 | 149 | 100 | 84 |
| Pore Volume (Hg, ml/g) | 0.77 | 0.77 | 0.84 | 0.57 | 0.44 |
| Particle diameter (mm) | 2.5 | 2.5 | 2.5 | 3.6 | 2.5 |
| Conversion (%) after 50 h | 99.9 | 99.9 | 98.0 | 99.7 | 99.9 |
| Selectivity to styrene (%) after 50 h | 96.5 | 96.5 | (a) | 95.9 | 95.0 |
| Selectivity to heavy ends (ethers + oligomers) (%) after 50 h | 3.0 | 3.0 | (a) | 3.5 | 4.3 |
| Conversion(%) after 120 h | 99.7 | 99.8 | (b) | 97.3 | (c) |

| | | | | | |
|---|---|---|---|---|---|
| (a) Not determined (b) Experiment stopped after 98 hours when conversion was 79% (c) Experiment stopped after 113 hours when conversion was 91% | | | | | |

## Claims

1. A process for the preparation of styrene comprising the gas phase dehydration of 1-phenylethanol at elevated temperature in the presence of a dehydration catalyst, in which the dehydration catalyst comprises shaped alumina catalyst particles having a surface area (BET) of from 80 to 140 m²/g and a pore volume (Hg) of more than 0.65 ml/g.

2. The process according to claim 1 wherein the catalyst has a pore volume (Hg) of from 0.75 to 0.85 ml/g.

3. The process according to claim 1 and/or 2 wherein the alumina catalyst is prepared from pseudo-boehmite.

## Patentansprüche

1. Verfahren zur Herstellung von Styrol, umfassend die Gasphasen-Dehydratisierung von 1-Phenylethanol bei erhöhter Temperatur in Gegenwart eines Dehydratisierungskatalysators, wobei der Dehydratisierungskatalysator geformte Aluminiumoxid-Katalysatorteilchen mit einer Oberflächenfläche (BET) von 80 bis 140 m²/g und einem Porenvolumen (Hg) von mehr als 0,65 ml/g umfasst.

2. Verfahren nach Anspruch 1, wobei der Katalysator ein Porenvolumen (Hg) von 0,75 bis 0,85 ml/g besitzt.

3. Verfahren nach Anspruch 1 und/oder 2, wobei der Aluminiumoxidkatalysator aus Pseudo-Böhmit hergestellt wird.

## Revendications

1. Procédé de préparation de styrène comprenant la déshydratation en phase gazeuse du 1-phényl-éthanol à température élevée en présence d'un catalyseur de déshydratation, dans lequel le catalyseur de déshydratation comprend des particules de catalyseur à base d'alumine mises en forme ayant une superficie (BET) de 80 m²/g à 140 m²/g et un volume de pore (Hg) supérieur à 0,65 ml/g.

2. Procédé selon la revendication 1, dans lequel le catalyseur a un volume de pore (Hg) de 0,75 ml/g à 0,85 ml/g.

3. Procédé selon la revendication 1 et/ou la revendication 2, dans lequel le catalyseur à base d'alumine est préparé à partir de pseudo-boehmite.
